(19) Europäisches Patentamt
European Patent Office
Office européen des brevets

(11) Veröffentlichungsnummer: **0 175 650**
**A2**

(12) # EUROPÄISCHE PATENTANMELDUNG

(21) Anmeldenummer: **85810418.5**

(22) Anmeldetag: **13.09.85**

(51) Int. Cl.⁴: **C 07 D 277/64**, C 07 D 263/56, C 07 D 417/04, A 61 K 31/425, A 61 K 31/42

(30) Priorität: **19.09.84 GB 8423697**

(43) Veröffentlichungstag der Anmeldung: **26.03.86 Patentblatt 86/13**

(84) Benannte Vertragsstaaten: **AT BE CH DE FR GB IT LI LU NL SE**

(71) Anmelder: **CIBA-GEIGY AG, Klybeckstrasse 141, CH-4002 Basel (CH)**

(72) Erfinder: **Rao, Vittal Ramachandra, Y-2/28 Government Colony Bandra East, Bombay 400051 (IN)**

(54) **Benzazole, Verfahren zu ihrer Herstellung und pharmazeutische Präparate enthaltend solche Verbindungen und deren Verwendung.**

(57) Die vorliegende Erfindung betrifft neue Benzazolderivate der allgemeinen Formel I

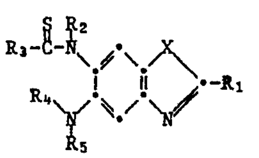

(I)

worin X Sauerstoff oder Schwefel, $R_1$ Niederalkyl, Niederalkenyl oder Cycloalkyl gegebenenfalls substituiert, $R_2$ Wasserstoff, und $R_3$ Niederalkylthio oder Niederalkenylthio gegebenenfalls substituiert durch eine veresterte Hydroxy-, Carboxy- oder veresterte Carboxygruppe oder $R_2$ und $R_3$ beide zusammengenommen eine zusätzliche Bindung in der C-N-Gruppierung, und $R_4$ und $R_5$ jeder für sich Wasserstoff, Niederalkyl oder Cycloalkyl oder beide zusammengenommen einen unsubstituierten oder substituierten bivalenten Kohlenwasserstoffrest aliphatischen Charakters, in welchem die Kohlenstoffatome der Kette durch ein Heteroatom unterbrochen sein können, bedeuten, ihre Salze, sowie Verfahren zu ihrer Herstellung, die Verbindungen der Formel I und ihre Salze als pharmakologisch aktive Verbindungen, pharmazeutische Präparate enthaltend solche Verbindungen und die Verwendung der neuen Verbindungen als pharmakologisch wirksame Substanzen, sowie zur Herstellung von pharmazeutischen Präparaten.



ACTORUM AG

CIBA-GEIGY AG
Basel (Schweiz)

4-15036/+/CIN 111

Benzazole, Verfahren zu ihrer Herstellung und pharmazeutische Präparate enthaltend solche Verbindungen und deren Verwendung

Die vorliegende Erfindung betrifft neue Benzazolderivate der allgemeinen Formel I

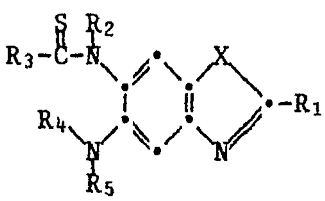

(I)

worin X Sauerstoff oder Schwefel, $R_1$ Niederalkyl, Niederalkenyl oder Cycloalkyl gegebenenfalls substituiert, $R_2$ Wasserstoff, und $R_3$ Niederalkylthio oder Niederalkenylthio gegebenenfalls substituiert durch eine veresterte Hydroxy-, Carboxy- oder veresterte Carboxygruppe oder $R_2$ und $R_3$ beide zusammengenommen eine zusätzliche Bindung in der C-N-Gruppierung, und $R_4$ und $R_5$ jeder für sich Wasserstoff, Niederalkyl oder Cycloalkyl oder beide zusammengenommen einen unsubstituierten oder substituierten bivalenten Kohlenwasserstoffrest aliphatischen Charakters, in welchem die Kohlenstoffatome der Kette durch ein Heteroatom unterbrochen sein können, bedeuten, ihre Salze, sowie Verfahren zu ihrer Herstellung, die Verbindungen der Formel I und ihre Salze als pharmakologisch aktive Verbindungen, pharmazeutische Präparate enthaltend solche Verbindungen und die Verwendung der neuen Verbindungen als pharmakologisch wirksame Substanzen, sowie zur Herstellung von pharmazeutischen Präparaten.

Vor- und nachstehend sind unter niederen Resten und Verbindungen beispielsweise solche zu verstehen, die bis und mit 7, insbesondere bis und mit 4, Kohlenstoffatome aufweisen. Niederalkyl- oder auch -alkenylreste können geradkettig oder verzweigt sein und können z.B. durch freie, veresterte oder verätherte Hydroxygruppen, wie Niederalkanoyloxy-, Niederalkoxy oder Niederalkenyloxygruppen, oder Halogenatome, sowie gegebenenfalls veresterte Carboxygruppen, wie z.B. Niederalkoxycarbonyl, beispielsweise Methoxy- oder Aethoxycarbonyl, oder Niederalkylthio, wie z.B. Methylthio, Aethylthio, n-Propylthio, Isopropyl- oder n-Butylthio oder Niederalkenylthio, wie z.B. Allyl- oder 2-Methylallylthio substituiert sein.

Halogenatome sind in erster Linie Fluor-, Chlor- oder Bromatome, können aber auch Jodatome sein.

Niederalkylgruppen sind z.B. vorzugsweise Methyl- sowie Aethyl-, n-Propyl-, Isopropyl-, n-Butyl-, Isobutyl-, sek.-Butyl-, tert.-Butyl-, Isopentyl-, Neopentyl-, n-Hexyl-, Isohexyl- oder n-Heptylgruppen. Niederalkenylgruppen sind z.B. die Allyl oder die 2-Methylallylgruppe. Substituierte Niederalkylgruppen sind beispielsweise die Trifluormethylgruppe oder eine gegebenenfalls veresterte Carboxymethyl-, z.B. Methoxycarbonylmethylgruppe.

Niederalkoxy ist z.B. Methoxy, Aethoxy, n-Propoxy, Isopropoxy, n-Butoxy oder n-Pentyloxy, und Niederalkenyloxy z.B. Vinyloxy oder Allyloxy.

Eine Cycloalkylgruppe ist in erster Linie ein monocyclischer Rest mit beispielsweise 3 bis 10, vorzugsweise 5 bis 7 Ringkohlenstoffatomen und ist z.B. eine Cyclopropyl-, Cyclobutyl-, vorzugsweise jedoch eine Cyclopentyl-, Cyclohexyl- oder Cycloheptylgruppe.

Die beiden Substituenten $R_4$ und $R_5$ zusammengenommen können auch einen gegebenenfalls substituierten bivalenten aliphatischen Kohlenwasserstoffrest mit 4-7 Kohlenstoffen in der Kette bedeuten.

Substituenten am bivalenten aliphatischen Kohlenwasserstoffrest sind beispielsweise ein oder auch mehrere Niederalkylreste oder auch ein gegebenenfalls substituierter Phenylrest.

Diese Gruppe bedeutet jedoch mit dem gemeinsamen Stickstoffatom auch Niederalkylenamino, in der die Niederalkylenkette beispielsweise durch ein Heteroatom, wie z.B. Sauerstoff, Schwefel oder gegebenenfalls substituierter Stickstoff unterbrochen sein kann, und stellt als Niederalkylenamino z.B. Pyrrolidino, Piperidino, 2-Methyl-, 4-Methyl- oder 4-Phenylpiperidino, Hexahydroazepino oder Octahydroazocino, als Oxaniederalkylenamino z.B. Morpholino, 2,6-Dimethylmorpholino, als Thioniederalkylenamino z.B. Thiomorpholino und als gegebenenfalls substituiertes Azaniederalkylenamino z.B. Piperazino, N-Methyl-, N-Phenyl-, N-Acetyl-, N-Methoxycarbonyl-, N-Aethoxycarbonyl- oder N-Methansulfonyl-piperazino, d.h. Substituenten an gegebenenfalls substituierten Stickstoffatomen des Azaniederalkylenrestes sind beispielsweise Niederalkyl, Aryl, wie z.B. Phenyl, Acyl, wie z.B. Niederalkanoyl, insbesondere Acetyl oder Aroyl, insbesondere Benzyl, oder Niederalkoxycarbonyl, wie z.B. Aethoxycarbonyl oder Methoxycarbonyl oder Niederalkansulfonyl, wie z.B. Methansulfonyl.

Phenylreste können gegebenenfalls durch Niederalkyl, Niederalkoxy, Niederalkoxycarbonyl oder auch Halogen substituiert sein.

Die neuen Verbindungen besitzen wertvolle pharmakologische Eigenschaften. Sie ermöglichen einen Fortschritt in der Behandlung parasitärer Helminthen wie z.B. Nematoden, Zestoden und Trematoden. Sie sind insbesondere für die Behandlung von Erregern (pathogenen) der Filariasis, wie z.B. Litomosoides carinii, Brugia malayi, Brugia pahangi und Dipetalonema viteae und allen ihren Entwicklungsstadien. Ferner sind sie geeignet für die Behandlung der Filariasis in der vielstreifigen Ratte (Mastomys natalensis) und Sandrennmaus (Meriones unguiculatus). Die neuen Verbindungen haben sich als sehr wirksame Makro und Mikrofilarizide bei 2-5-facher oraler Verabreichung in Dosen von 5 bis 25 mg/kg erwiesen.

Die Erfindung betrifft in erster Linie Verbindungen der allgemeinen Formel I, in der X Sauerstoff oder Schwefel, $R_1$ Niederalkyl oder Niederalkenyl, $R_2$ Wasserstoff, und $R_3$ gegebenenfalls durch Carboxy- oder veresterte Carboxygruppe substituiertes Niederalkylthio, oder $R_2$ und $R_3$ zusammengenommen eine zusätzliche Bindung in der C-N-Gruppierung, und $R_4$ und $R_5$ jeder für sich Wasserstoff oder Niederalkyl oder beide zusammengenommen einen unsubstituierten oder substituierten Niederalkylenrest, der gegebenenfalls durch ein Sauerstoff-, Schwefel- oder gegebenenfalls substituiertes Stickstoffatom unterbrochen sein kann mit 4-6 Kohlenstoffatomen in der Kette bedeuten und ihre Salze.

Von besonderer Bedeutung sind Verbindungen der allgemeinen Formel I, in der X Schwefel, $R_1$ Niederalkyl, $R_2$ Wasserstoff und $R_3$ eine Carboxyniederalkylthiogruppe oder $R_2$ und $R_3$ zusammengenommen eine zusätzliche Bindung in der C-N-Gruppierung, und $R_4$ und $R_5$ jeder für sich Wasserstoff, Niederalkyl oder beide zusammen einen gegebenenfalls durch ein Sauerstoff-, Schwefel- oder Stickstoffatom, welches gegebenenfalls durch Niederalkyl, Niederalkanoyl, Niederalkoxycarbonyl, Niederalkansulfonyl oder Phenyl substituiert sein kann, unterbrochen sein kann, bedeuten und ihre Salze.

Von besonderem Interesse sind Verbindungen der Formel Ia

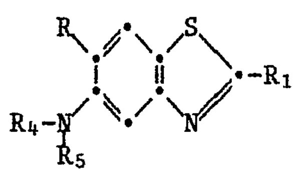

(Ia)

worin R die NCS oder $HN-\overset{S}{\overset{\|}{C}}-S(CH_2)_2COOH$ Gruppe, $R_1$ Niederalkyl mit 3 bis 4 C-Atomen und $R_4$ und $R_5$ jeder für sich Wasserstoff, Niederalkyl oder beide zusammen mit dem benachbarten Stickstoffatom einen Niederalkylenaminorest mit 4 oder 5 Kohlenstoffatomen in der Kette, der gegebenenfalls durch ein Sauerstoff-, Schwefel- oder auch gegebenenfalls durch Niederalkyl, Niederalkanoyl, Niederalkoxycarbonyl, Niederalkansulfonyl substituierten Stickstoffatom unterbrochen sein kann, bedeuten und ihre pharmazeutisch verwendbaren Salze.

Von ganz besonderem Interesse sind Verbindungen der Formel Ia worin R die NCS oder $HN-\underset{\underset{S}{\|}}{C}-S(CH_2)2-COOH$ Gruppe, $R_1$ tert-Butyl und $R_4$ und $R_5$ Niederalkyl, wie z.B. Methyl oder beide zusammen mit dem benachbarten Stickstoffatom als Niederalkylenamino einen heterocyclischen Rest, wie z.B. Pyrrolidino, Piperidino, Morpholino, Piperazino oder Methylpiperazino, Phenyl-piperazino, Acetylpiperazino, Methansulfonyl-piperazino, Methoxycarbonyl- oder Aethoxycarbonyl-piperazino bedeuten und ihre pharmazeutisch verwendbaren Salze.

Besonders zu nennen sind die in den Beispielen beschriebenen Verbindungen der Formel I und ihre Salze, insbesondere ihre pharmazeutisch verwendbaren nicht-toxischen Salze.

Die neuen Benzimidazole der Formel I werden nach an sich bekannten Methoden erhalten.

Die neuen Verbindungen der Formel I können erhalten werden, indem man Verbindungen der Formel II

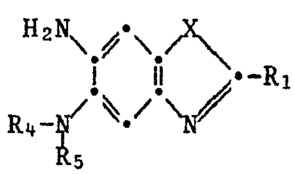

(II)

worin X, $R_1$, $R_4$ und $R_5$ die unter der Formel I angegebenen Bedeutungen haben mit Thiophosgen in einem inerten Lösungsmittel behandelt.

Nach einem zweiten Verfahren können Isothiocyanobenzazole der Formel I erhalten werden, indem man Verbindungen der Formel III

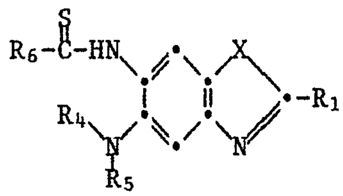

(III)

worin X, $R_1$, $R_4$ und $R_5$ die unter der Formel I angegebene Bedeutung haben und $R_6$ eine durch Pyrolyse abspaltbare Gruppe, vorzugsweise Amino, Niederalkylthio oder die Thioammoniumgruppe $^{\oplus}NH_4-\overset{\ominus}{S}-$ bedeutet, pyrolysiert. Als besonderes Verfahrensmerkmal führt man die Pyrolyse beispielsweise in einem hochsiedenden Lösungsmittel, wie z.B. siedendem Chlorbenzol, aus.

Eine Variante des oben erwähnten Verfahrens ist die oxydative Pyrolyse, welche beispielsweise durch Oxydation von Verbindungen der Formel III, worin $R_6$ eine Thioammoniumgruppe $^{\oplus}NH_4-\overset{\ominus}{S}-$ bedeutet und X, $R_1$, $R_4$ und $R_5$ oben angegebenen Bedeutungen haben, erfolgt. Man führt die Oxydation beispielsweise mit Hilfe von Oxydationsmitteln, wie z.B. Bleinitrate oder Natriumhypochlorit aus.

Isothiocyanobenzazole der Formel I können auch nach einem dritten Verfahren erhalten werden, indem man Verbindungen der Formel IV

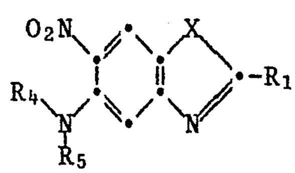

(IV)

worin X, $R_1$, $R_4$ und $R_5$ die oben angegebenen Bedeutungen haben, mit Schwefelkohlenstoff (Carbondisulfid), vorzugsweise in Gegenwart von Natriumhydroxid in einen Autoklaven gemäss G. Ottmann und E. Kober, Angew. Chemie. Int. Bd. 8, 780 (1969) behandelt.

Nach einem vierten Verfahren können Isothiocyanobenzazole der Formel I erhalten werden, indem man Verbindungen der Formel V

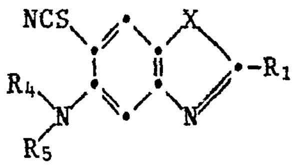

(V)

worin X, $R_1$, $R_4$ und $R_5$ die oben angegebenen Bedeutungen haben in einem hochsiedenden Lösungsmittel thermisch isomerisiert.

Die erhaltenen Verbindung der allgemeinen Formel I, worin $R_2$ und $R_3$ beide zusammen eine zusätzliche Bindung in der C-N Gruppierung bedeuten, können auf übliche Weise in Verbindungen der Formel I, in der $R_2$ Wasserstoff und $R_3$ beispielsweise eine Carboxyalkylthio-Gruppe bedeuten, umgewandelt werden. So können Benzazoldithiocarbamate der Formel I erhalten werden, worin $R_2$ Wasserstoff und $R_3$ Niederalkylthio bedeuten, in dem man Isothiocyanobenzazole der Formel I mit ω-Mercaptoalkanen der Formel VI

$$R\text{-}(CH_2)_n\text{-}SH \qquad (VI)$$

worin n eine integere Zahl von 2 oder 3 und R eine Carboxygruppe, Niederakoxycarbonyl oder Niederalkoxy bedeuten.

Benzazoldithiocarbamate der Formel I können auch wie oben gemäss dem ersten Verfahren aus Verbindungen der Formel II nach Behandlung mit Schwefelkohlenstoff in Gegenwart von einem Alkalimetall und nachträglicher Behandlung mit einer ω-Halogenalkylcarbonsäure, einem -ester oder -äther, wie z.B. ω-Bromalkylcarbonsäure, -ester oder -äther erhalten werden.

Die beschriebenen Verfahren können in gewohnter Weise bei Raumtemperatur, unter Kühlen oder Erwärmen, bei Normaldruck oder erhöhtem Druck, und, wenn notwendig, in Gegenwart oder Abwesenheit eines Verdünnungsmittels, Katalysators oder Kondensationsmittels ausgeführt werden. Wenn notwendig, können die Umsetzungen auch in der

Atmosphäre eines inerten Gases, wie z.B. Stickstoff erfolgen. In erhaltenen Verbindungen kann man im Rahmen der Definition der Endprodukte Substituenten einführen, abwandeln oder abspalten.

Je nach den Verfahrensbedingungen und Ausgangsstoffen erhält man die Endstoffe in freier Form oder in der ebenfalls in der Erfindung inbegriffenen Form ihrer Salze, insbesondere Säureadditionssalze. Die Säureadditionssalze der neuen Verbindung können in an sich bekannter Weise in die freie Verbindung übergeführt werden, z.B. mit basischen Mitteln, wie Alkalien oder Ionenaustauschern. Andererseits können die erhaltenen freien Basen mit organischen oder anorganischen Säuren Salze bilden. Zur Herstellung von Säureadditionssalzen werden insbesondere solche Säuren verwendet, die zur Bildung von therapeutisch verwendbaren Salzen geeignet sind. Als solche Säuren seien beispielsweise genannt: Halogenwasserstoffsäuren, Schwefelsäuren, Phosphorsäuren, Salpetersäure, Perchlorsäure, aliphatische, alicyclische, aromatische oder heterocyclische Carbon- oder Sulfonsäuren, wie Ameisen-, Essig-, Propion-, Bernstein-, Glykol-, Milch-, Aepfel-, Wein-, Zitronen-, Ascorbin-, Malein-, Hydroxymalein- oder Brenztraubensäure; Phenylessig-, Benzoe-, p-Aminobenzoe-, Anthranil-, p-Hydroxybenzoe-, Salicyl- oder p-Aminosalicylsäure, Embonsäure, Methansulfon-, Aethansulfon-, Hydroxyäthansulfon-, Aethylensulfonsäure; Halogenbenzolsulfon-, Toluolsulfon-, Naphthalinsulfonsäure oder Sulfanilsäure; Methionin, Trypthophan, Lysin oder Arginin.

Diese oder andere Salze der neuen Verbindungen, wie z.B. Pikrate, können auch zur Reinigung der erhaltenen freien Basen dienen, indem man die freien Basen in Salze überführt, diese abtrennt und aus den Salzen wiederum die Basen freimacht. Infolge der engen Beziehungen zwischen den neuen Verbindungen in freier Form und in Form ihrer Salze sind im Vorausgegangenen und nachfolgend unter den freien Verbindungen sinn- und zweckmässig, gegebenenfalls auch die entsprechenden Salze zu verstehen.

Die Erfindung betrifft auch diejenigen Ausführungsformen eines Verfahrens, bei denen man ein Verfahren auf irgendeiner Stufe abbricht oder bei denen man von einer auf irgendeiner Stufe als Zwischenprodukt erhältlichen Verbindung ausgeht und die fehlenden Schritte durchführt, oder einen Ausgangsstoff unter den Reaktionsbedingungen bildet oder gegebenenfalls in Form eines Salzes verwendet. Die Erfindung beinhaltet auch daraus resultierende neue Zwischenprodukte.

Bei den Verfahren der vorliegenden Erfindung werden vorzugsweise solche Ausgangsstoffe verwendet, welche zu den eingangs als besonders wertvoll geschilderten Verbindungen führen. Neue Ausgangsstoffe und Verfahren zu ihrer Herstellung bilden ebenfalls einen Gegenstand der Erfindung.

Ausgangsverbindungen der Formel II lassen sich in einer Folge von bekannten Reaktionsschritten ausgehend von 2,5-Dichloranilin herstellen. Durch Acylierung mit einem Alkancarbonsäurechlorid erhält man das entsprechende Amid, welches nach Nitrierung und darauffolgender Behandlung mit Phosphorpentasulfid ein Thioamid der Formel VIII

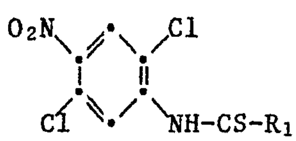

(VIII)

worin $R_1$ die oben unter der Formel I angegebenen Bedeutungen hat, ergibt.

Durch Behandlung der erhaltenen Verbindungen der Formel VIII mit Triäthylamin in Dimethylsulfoxid und nachträglicher Behandlung mit

einem acyclischen oder cyclischen sekundären Amin erhält man Verbindungen der Formel IX

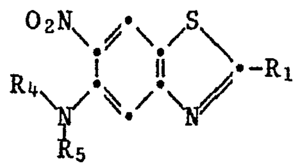

(IX)

worin $R_1$, $R_4$ und $R_5$ die unter der Formel I angegebenen Bedeutungen haben. Verbindungen der Formel IX können jedoch auch aus Verbindungen der Formel VIII in einer Stufe durch Verwendung eines Ueberschusses an einem acyclischen oder cyclischen sekundären Amins in Dimethylsulfoxyd in Gegenwart von Kaliumcarbonat erhalten werden. Die Reduktion der anorganischen Nitroverbindungen der Formel IX zu Verbindungen der Formel II wird in an sich bekannter Weise durch Hydrierung in Gegenwart von Raney Nickel oder eines Paladium-auf-Kohle-Katalysators in einem geeigneten Lösungsmittel ausgeführt. Diese Reduktion kann jedoch auch nach bekannten chemischen Verfahren, beispielsweise unter Verwendung von Metallen, wie z.B. Zink oder Eisen in Gegenwart einer Mineralsäure oder Alkancarbonsäure, wie z.B. Essigsäure ausgeführt werden.

Die Ausgangsverbindungen der Formel III des zweiten Verfahrens können aus Verbindungen der Formel II in bekannter Weise erhalten werden. Beispielsweise erhält man Verbindungen der Formel III, worin $R_6$ eine Thioammoniumgruppe bedeutet, durch Umsetzung einer Verbindung der Formel II mit Ammoniumthiocyanat in Gegenwart von konzentrierter Schwefelsäure unter Rückflusstemperatur.

Die Ausgangsverbindungen der Formel V können hergestellt werden, indem man in Verbindungen der Formel X

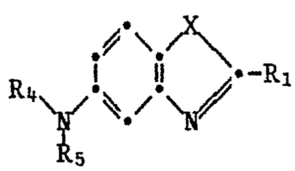

(X)

worin X, $R_1$, $R_4$ und $R_5$ die oben angegebenen Bedeutungen haben, die Thiocyanatgruppe gemäss einer in der Literatur (E.D.Sych.Ukrain.Khim.Zhur, 25, 767-73 (1958), C.A. 54, 13144) beschriebenen Methode einführt.

Evaluierungen in tierischen oben erwähnten Testsystemen zeigen die angezeichnete Eigenschaft der neuen Verbindungen der Formel I, diese als Micro- und Macrofilarizide, beispielsweise für die lymphatische Filariasis, Orchocerciasis und Schistosomiasis in wirksamen Tagesdosen zwischen 5 und 50 mg/kg bei oraler Gabe über eine Zeitperiode von 1 bis 5 Tagen zu verwenden.

Die Erfindung betrifft im weiteren pharmazeutische Präparate, die Verbindungen der allgemeinen Formel I als Wirkstoffe enthalten, sowie Verfahren zur Herstellung.

Die erfindungsgemässen pharmazeutischen Präparate enthalten mindestens eine Verbindung der allgemeinen Formel I oder ein Salz davon als Wirkstoff zusammen mit einem üblichen pharmazeutischen Trägerstoff. Die Art der Trägerstoffe richtet sich weitgehend nach dem Anwendungsgebiet. Die erfindungsgemässen pharmazeutischen Stoffzusammensetzungen, die als Wirkstoffe Verbindungen der Formel I enthalten, können oral, parenteral oder rektal verabreicht werden.

Zur oralen Behandlung von parasitären Infektionen kommen insbesondere feste Doseneinheitsformen, wie Tabletten, Dragées und Kapseln, in Frage, die vorzugsweise zwischen 10 und 90 % eines Wirkstoffes der allgemeinen Formel I oder eines Salzes enthalten, um die Verabreichung von täglichen Dosen zwischen 5 bis 50 mg/kg an Warmblütern zu ermöglichen. Zur Herstellung von Tabletten und Dragée-Kernen kombiniert man die Verbindungen der allgemeinen Formel I mit festen, pulverförmigen Trägerstoffen, wie Lactose, Saccharose, Sorbit, Maisstärke, Kartoffelstärke oder Amylopektin, Cellulosederivaten oder Gelatine, vorzugsweise unter Zusatz von Gleitmitteln, wie Magnesium- oder Calciumstearat, oder Polyäthylenglykolen von geeignetem Molekulargewicht. Dragéekerne überzieht man

anschliessend beispielsweise mit konzentrierten Zuckerlösungen, welche z.B. noch arabischen Gummi, Talk und/oder Titandioxid enthalten können, oder mit einem in leichtflüchtigen organischen Lösungsmitteln oder Lösungsmittelgemischen gelösten Lack. Diesen Ueberzügen können Farbstoffe zugefügt werden, z.B. zur Kennzeichnung verschiedener Wirkstoffdosen. Weiche Gelatinekapseln und andere geschlossene Kapseln bestehen beispielsweise aus einem Gemisch von Gelatine und Glycerin und können z.B. Mischungen einer Verbindung der Formel I mit Polyäthylenglykol enthalten. Steckkapseln enthalten z.B. Granulate eines Wirkstoffes mit festen, pulverförmigen Trägerstoffen, wie z.B. Lactose, Saccharose, Sorbit, Mannit; Stärken, wie Kartoffelstärke, Maisstärke oder Amylopektin, Cellulosederivate und Gelatine sowie Magnesiumstearat oder Stearinsäure.

Als Doseneinheitsformen für die rektale Anwendung kommen z.B. Suppositorien in Betracht, welche aus einer Kombination eines Wirkstoffes mit einer Suppositorien-Grundmasse auf der Basis von natürlichen oder synthetischen Triglyceriden (z.B. Kakaobutter), Polyäthylenglykolen oder geeigneten höheren Fettalkoholen bestehen, und Gelatine-Rektalkapseln, welche eine Kombination des Wirkstoffes mit Polyäthylenglykolen enthalten.

Ampullenlösungen zur parenteralen, insbesondere intramuskulären oder intravenösen Verabreichung enthalten eine Verbindung der Formel I oder ein Salz davon in einer Konzentration von vorzugsweise 0,5 bis 5 % als wässrige, mit Hilfe von üblichen Lösungsvermittlern und/oder Emulgiermitteln, sowie gegebenenfalls von Stabilisierungsmitteln bereitete Dispersion, oder vorzugsweise eine wässrige Lösung eines pharmazeutisch annehmbaren, wasserlöslichen Säureadditionssalzes einer Verbindung der allgemeinen Formel I.

Für Flüssigkeiten zur oralen Einnahme, wie Sirups und Elixiere, wird die Konzentration des Wirkstoffes derart gewählt, dass eine Einzeldosis leicht abgemessen werden kann, z.B. als Inhalt eines Teelöffels oder eines Messlöffels von z.B. 5 ml, oder auch als Mehrfaches dieser Volumina.

Die nachfolgenden Beispiele a) bis e) sollen die Herstellung einiger typischer Applikationsformen erläutern, jedoch keineswegs die einzigen Ausführungsformen von solchen darstellen.

a) 250,0 g Wirkstoff werden mit 550,0 g Lactose und 292,0 g Kartoffelstärke vermischt, die Mischung mit einer alkoholischen Lösung von 8 g Gelatine befeuchtet und durch ein Sieb granuliert. Nach dem Trocknen mischt man 60,0 g Talk, 10,0 g Magnesiumstearat und 20,0 g kolloidales Siliciumdioxid zu und presst die Mischung zu 10'000 Tabletten von je 125 mg Gewicht und 25 mg Wirkstoffgehalt, die gewünschtenfalls mit Teilkerben zur feineren Anpassung der Dosierung versehen sein können.

b) Aus 100,0 g Wirkstoff, 379,0 g Lactose und der alkoholischen Lösung von 6,0 g Gelatine stellt man ein Granulat her, das man nach dem Trocknen mit 10,0 g kolloidalem Siliciumdioxid, 40,0 g Talk, 60,0 g Kartoffelstärke und 5,0 g Magnesiumstearat mischt und zu 10'000 Dragée-Kernen presst. Diese werden anschliessend mit einem konzentrierten Sirup aus 533,5 g krist. Saccharose, 20,0 g Schellack, 75,0 g arabischem Gummi, 250,0 g Talk, 20,0 g kolloidalem Siliciumdioxid und 1,5 g Farbstoff überzogen und getrocknet. Die erhaltenen Dregées wiegen je 150 mg und enthalten je 10 mg Wirkstoff.

c) 25,0 g Wirkstoff und 1975 g fein geriebene Suppositoriengrundmasse (z.B. Kakaobutter) werden gründlich gemischt und dann geschmolzen. Aus der durch Rühren homogen gehaltenen Schmelze werden 1000 Suppositorien von 2,0 g gegossen. Sie enthalten je 25 mg Wirkstoff.

d) Zur Bereitung eines Sirups mit 0,25 % Wirkstoffgehalt löst man in 3 Litern dest. Wasser 1,5 Liter Glycerin, 42 g p-Hydroxybenzoesäure-methylester, 18 g p-Hydroxybenzoesäure-n-propylester und unter leichtem Erwärmen 25,0 g Wirkstoff fügt 4 Liter 70%-ige Sorbitlösung, 1000 g krist. Saccharose, 350 g Glucose und einen Aromastoff,

z.B. 250 g "Orange Peel Soluble Fluid" von Eli Lilly und Co., Indianapolis, oder je 5 g natürliches Zitronenaroma und 5 g "Halb und Halb"-Essenz, beide von der Firma Haarmann und Reimer, Holzminden, Deutschland, zu, filtriert die erhaltene Lösung und ergänzt das Filtrat mit dest. Wasser auf 10 Liter.

e) Zur Bereitung einer Tropflösung mit 1,5 % Wirkstoffgehalt löst man 150,0 g Wirkstoff und 30 g Natriumcyclamat in einem Gemisch von 4 Liter Aethanol (96 %) und 1 Liter Propylenglykol. Andererseits mischt man 3,5 Liter 70%-ige Sorbitlösung mit 1 Liter Wasser und fügt die Mischung zur obigen Wirkstofflösung. Hierauf wird ein Aromastoff, z.B. 5 g Hustenbonbon-Aroma oder 30 g Grapefruit-Essenz, beide von der Firma Haarmann und Reimer, Holzminden, Deutschland, zugegeben, das Ganze gut gemischt, filtriert und mit dest. Wasser auf 10 Liter ergänzt.

Die nachfolgenden Beispiele erläutern die Herstellung der neuen Verbindungen der allgemeinen Formel I, sollen jedoch den Umfang der Erfindung in keiner Weise beschränken. Die Temperaturen sind in Celsiusgraden angegeben.

<u>Beispiel 1:</u> Ein Gemisch aus 100 g N-[2-Tert-butyl-5-(piperidin-1-yl)]benzothia-6-yl-harnstoff und 18 g Ammoniumsulfat in 3000 ml getrocknetem Chlorbenzol wird 8 Stunden lang zum Rückfluss erhitzt und der entstehende frei werdende Ammoniak wird mit Hilfe eines Stickstoffstromes entfernt. Nach dem Abkühlen wird das Lösungsmittel aus dem Reaktionsgemisch entfernt und mit Hexan extrahiert. Der Hexanextrakt wird durch ein Filterbett, enthaltend 200 g Silicagel, filtriert und das erhaltene Filtrat wird eingedampft, wobei 2-Tert-butyl-6-isothiocyanato-5-[piperidin-1-yl]benzothiazol

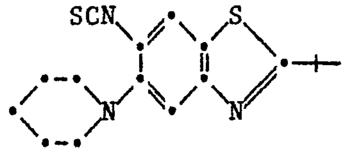

mit einem Schmelzpunkt von 112-114°C anfällt.

Das Ausgangsmaterial wird wie folgt hergestellt:

Zu einer gerührten Lösung von 1 kg 2,5-Dichloranilin in 2500 ml Pyridin werden 840 ml Pivaloylchlorid hinzugegeben und das Gemisch wird 4 Stunden lang zum Rückfluss erhitzt. Nach dem Abkühlen wird das Reaktionsgemisch in Wasser geschüttelt. Der feste Rückstand wird abfiltriert, mit Wasser gewaschen und an der Luft getrocknet. Man erhält N-Tert-butylcarbonyl-2,5-dichloranilin mit einem Schmelzpunkt von 74-76°.

Zu einem gerührten Gemisch von 369 g der oben erhaltenen Verbindung in 1800 ml konz. Schwefelsäure wird ein Gemisch von 465 ml konz. Salpetersäure und 230 ml Wasser bei -2° über ein Zeitintervall von 1 1/2 Stunden zugetropft. Das hierbei erhaltene Gemisch wird eine weitere halbe Stunde bei -2° gerührt und anschliessend auf Eiswasser aufgegossen. Der feste Rückstand wird abfiltriert, in Wasser suspendiert und mit verdünnter Natriumbicarbonatlösung bis zum pH 7 versetzt. Der erhaltene feste Rückstand wird abfiltriert und mit Wasser und kaltem Alkohol gewaschen. Man erhält N-Tert-butylcarbonyl-2,5-dichlor-4-nitroanilin mit einem Schmelzpunkt von 142-145°.

Ein Gemisch aus 300 g der oben erhaltenen Verbindung und 360 g Phosphorpentasulfid in 2500 ml Azetonitril wird unter Rühren 15 Stunden lang zum Rückfluss erhitzt, anschliessend abgekühlt und filtriert. Das Filtrat wird im Vakuum zur Trockne eingeengt und der erhaltene Rückstand aus Alkohol umkristallisiert. Man erhält das N-Tert-butylthiocarbonyl-2,5-dichlor-4-nitroanilin mit einem Schmelzpunkt von 142-144°.

Unter Rühren wird ein Gemisch aus 462 g der erhaltenen Verbindung, 421 g getrocknetem Kaliumcarbonat und 225 ml Piperidin in 1200 ml Dimethylsulfoxid 10 Stunden lang auf 150° erhitzt, gekühlt und anschliessend in Wasser geschüttet. Der feste Rückstand wird

abfiltriert, mit Wasser und Isopropylalkohol gewaschen. Man erhält 2-Tertbutyl-6-nitro-5-[piperidin-1-yl]benzothiazol mit einem Schmelzpunkt von 147-148°.

Zu einer ausgiebig gerührten siedenden Suspension aus 560 g aktiviertem Eisen in 500 ml destilliertem Wasser, welches 66 ml Essigsäure enthält, werden unter Stickstoffathmosphäre 252 g der oben erhaltenen Verbindung in Portionen zugegeben und das erhaltene Gemisch weitere 10 Stunden lang zum Rückfluss erhitzt. Nach dem Abkühlen wird das Gemisch filtriert und der feste Rückstand mit Aethylazetat extrahiert. Der erhaltene Aethylazetatrückstand wird eingeengt, mit Hexan behandelt und man erhält das 6-Amino-2-tert-butyl-5-[piperidin-1-yl]benzothiazol mit einem Schmelzpunkt von 172-175°.

Unter Rühren wird ein Gemisch aus 288 g der erhaltenen Verbindung und 150 g getrockneten Isothiocyanat in 1500 ml Isopropylalkohol auf 60° unter Stickstoffatmosphäre erhitzt und 100 g konz. Schwefelsäure werden tropfenweise hinzugefügt und das erhaltene Reaktionsgemisch wird anschliessend 12 Stunden lang unter Rückfluss erhitzt, gekühlt und anschliessend in Wasser geschütttet. Die Suspension wird mit einer 10%igen Natronlauge bis zu einem pH von 8 behandelt. Der feste Rückstasnd wird abfiltriert, mit Wasser und Methanol gewaschen. Man erhält den N-[2-Tert-butyl-5-(piperidin-1-yl)benzothiazol-6-yl]thioharnstoff, Schmelzpunkt 228-232°.

<u>Beispiel 2:</u> Eine Lösung aus 1 g 2-Tert-butyl-6-isothiocyanat-5-[piperidin-1-yl]benzothiazol (beschrieben in Beispiel 1) und 0,5 g 3-Mercaptopropionsäure in 20 ml N,N-Dimethylformamid wird 24 Stunden lang unter Stickstoffatmosphäre bei Zimmertemperatur gerührt. Das erhaltene Lösungsgemisch in Wasser geschüttet, der feste Rückstand abfiltriert, mit Wasser gewaschen und aus Acetonitril umkristallisiert. Man erhält das 2-Tert-butyl-6-N-[(β-carboxyäthylthio)thiocarbonyl]-amino-5-[piperidin-1-yl]benzothiazol mit einem Schmelzpunkt von 158°.

Beispiel 3: Zu einem gerührten Gemisch aus 75 g 6-Amino-2-tert-butyl-5-[4-methylpiperazin-1-yl]benzothiazol und 29 g Natriumbicarbonat in 1100 ml Chloroform werden bei 0° 41 g Thiophosgen hinzugefügt und das Gemisch wird weitere 4 Stunden lang bei 10° gerührt. Nach dem Abfiltrieren des festen Rückstandes wird die Lösung eingeengt bis man einen gelben festen Rückstand erhält, der in Wasser gelöst wird und anschliessend der pH der Lösung auf 7 durch Hinzugabe von verdünnter Natronlauge eingestellt. Der feste Rückstand wird getrennt, mit Hexan extrahiert und durch 150 g neutrales Aluminiumoxid filtriert. Man erhält 2-Tert-butyl-6-isocyanat-5-[4-methyl-piperazin-1-yl]benzothiazol mit einem Schmelzpunkt von 124-126°.

Das Ausgangsmaterial der oben erhaltenen Verbindung wird wie folgt hergestellt:

Eine Lösung von 171 g N-Tert-butylthiocarbonyl-2,5-dichlor-4-nitroanilin (beschrieben in Beispiel 1) und 605 ml N-Methylpiperazin in 1700 ml Dimethylsulfoxid werden 8 Stunden lang auf 140° erhitzt, gekühlt und anschliessend in Wasser geschüttet. Der feste Rückstand wird abfiltriert, mit Wasser und kaltem Isopropanol gewaschen, wobei man 2-Tert-butyl-5-(4-methyl-piperazin-1-yl)-6-nitrobenzothiazol mit einem Schmelzpunkt von 132-133 erhält.

Eine Lösung von 86 g der erhaltenen Verbindung in 150 ml Aethanol wird in Gegenwart von 30 g Raney Nickel bei 45° hydriert. Die Lösung wird filtriert, um den Katalysator zu entfernen, eingeengt und der feste Rückstand abfiltriert. Man erhält 6-Amino-2-tert-butyl-5-[4-methyl-piperazin-1-yl]benzothiazol mit einem Schmelzpunkt von 130°.

Beispiel 4: Eine Lösung von 0,5 g 2-Tert-butyl-6-isothiocyanat-5-[4-methylpiperazin-1-yl]benzothiazol (beschrieben in Beispiel 3) und 0,25 g 3-Mercaptopropionsäure in 5 ml Dimethylformamid wird in einer Stickstoffatmosphäre 24 Stunden lang gerührt. Das Lösungsmittel wird unter Vakuum entfernt und der feste Rückstand wird aus Acetonitril

umkristallisiert. Man erhält das 2-Tert-butyl-6-N-[(β-carboxyäthylthio)thiocarbonyl]-amino-5-[4-methylpiperazin-1-yl]benzothiazol mit einem Schmelzpunkt von 167-170°.

Beispiel 5: Ein Gemisch aus 2,3 g N-2-[Tert-Butyl-5-(pyrrolidin-1-yl)benzothiazol-6-yl]thioharnstoff und eine Spatelspitze Ammoniumsulfat in 100 ml Chlorbenzol wird 6 Stunden lang unter einem Stickstoffatom zum Rückfluss erhitzt. Das Lösungsmittel Chlorbenzol wird im Vakuum entfernt und der Rückstand wird in Hexan gelöst und durch ein Silicagelfilter filtriert. Das erhaltene Filtrat wird eingeengt, wobei man das 2-Tert-butyl-6-isothiocyanat-5-[pyrrolidin-1-yl]benzothiazol mit einem Schmelzpunkt von 89-90° erhält.

Das Ausgangsmaterial für die obige Verbindung wird wie folgt erhalten:

Ein Gemisch aus 94 g N-Tert-butylthiocarbonyl-2,5-dichlor-4-nitroanilin (beschrieben im Beispiel 1) in 360 ml Dimethylsulfoxid und 160 ml Triäthylamin wird 10 Stunden lang unter Stickstoffatmosphäre auf 140° erhitzt. Das erhaltene Reaktionsgemisch wird gekühlt und in Wasser geschüttet. Der ausgefallene feste Rückstand wird abfiltriert, mit Wasser gewaschen und an der Luft getrocknet. Man erhält das 2-Tert-butyl-5-chlor-6-nitrobenzothiazol mit einem Schmelzpunkt von 65-69°.

Eine Lösung enthaltend 7 g der oben beschriebenen Verbindung in 15 ml Pyrrolidin wird 10 Stunden lang am Rückfluss erhitzt. Das Reaktionsgemisch wird im Vakuum eingeengt und der erhaltene Rückstand mit Alkohol ausgekocht. Nach dem Abkühlen erhält man das 2-Tert-butyl-6-nitro-5-[pyrrolidin-1-yl]benzothiazol mit einem Schmelzpunkt von 115-117.

Eine Lösung aus 6 g der oben erhaltenen Verbindung in 300 ml Methanol wird in Gegenwart von 3 g Raney Nickel hydriert. Der Katalysator wird abfiltriert, die Lösung eingeengt und der feste Rückstand abfiltriert. Man erhält 6-Amino-2-tert-butyl-5-[pyrrolidin-1-yl]benzothiazol mit einem Schmelzpunkt von 130-132°.

Ein Gemisch aus 4,8 g der erhaltenen Verbindung und 4,0 g Ammoniumthiocyanat in 20 ml Isopropylalkohol wird unter Stickstoffatmosphäre auf 60° erhitzt und anschliessend mit 2,5 g konz. Schwefelsäure tropfenweise versetzt. Das Reaktionsgemisch wird danach 7 Stunden lang zum Rückfluss erhitzt, abgekühlt, anschliessend mit Natronlauge stark basisch gestellt und filtriert. Der erhaltene feste Rückstand wird mit Wasser und Methanol gewaschen. Man erhält den N-[2-Tert-butyl-5-(pyrrolidin-1-yl)benzothiazol-6-yl]thioharnstoff, Schmelzpunkt 221°.

Beispiel 6: Zu einem gekühlten Gemisch von 143 g 6-Amino-2-tert-butyl-5-morpholino-benzothiazol und 168 g Natriumbicarbonat in 200 ml Chloroform werden 75 g Thiophosgen unter Rühren hinzugefügt. Das Rühren wird weitere 10 Stunden lang bei Zimmertemperatur festgesetzt. Die festen Bestandteile werden abfiltriert und das Filtrat wird eingeengt. Der erhaltene feste Rückstand wird in Hexan gelöst und eine Silicagelkolonne filtriert. Das Filtrat wird im Vakuum eingeengt, wobei man das 2-Tert-butyl-6-isothiocyanat-5-[morpholin-4-yl]benzothiazol mit einem Schmelzpunkt von 141-143° erhält.

Das Ausgangsmaterial wird wie folgt hergestellt:

Eine Lösung aus 170 g 2-Tert-butyl-5-chlor-6-nitrobenzothiazol (beschrieben in Beispiel 5) in 200 ml Morpholin wird 6 1/2 Stunden lang zum Rückfluss erhitzt. abgekühlt und in Wasser geschüttet. Der ausgefallene feste Rückstand wird abfiltriert, mit Wasser und Alkohol gewaschen. Man erhält das 2-Tert-butyl-5-(morpholin-4-yl)-6-nitro-benzothiazol mit einem Schmelzpunkt von 119-121°.

Eine Lösung aus 173 g der oben erhaltenen Verbindung in 5000 ml Methanol wird in Gegenwart von 40 g Raney Nickel bei Raumtemperatur hydriert. Nach dem Entfernen des Katalysators wird die Lösung eingeengt und der abfiltrierte feste Rückstand ergibt das 6-Amino-2-tert-butyl-5-morpholino-benzothiazol mit einem Schmelzpunkt von 158-159°.

Beispiel 7: Ein Gemisch aus 100 g 2-Tert-butyl-6-isothiocyanat-5-[morpholino-4-yl]benzothiazol (beschrieben in Beispiel 6) und 62,4 g 3-Mercaptopropionsäure in 700 ml N,N-Dimethylformamid wird unter Stickstoffatmosphäre 15 Stunden lang gerührt. Die klare Lösung wird in Wasser geschüttet, der feste ausgefallene Rückstand wird abfiltriert, mit Wasser gewaschen, an der Luft getrocknet und aus Aethylazetat umkristallisiert. Man erhält das 2-Tert-butyl-6-N-[(β-carboxyäthylthio)thiocarbonyl]-amino-5-[morpholin-4-yl]-benzothiazol mit einem Schmelzpunkt von 180.5-182.5°.

Beispiel 8: Zu einem Gemisch aus 2 g 6-Amino-2-tert-butyl-5-[thiomorpholin-4-yl]benzothiazol und 2 g getrocknetem Kaliumcarbonat in 150 ml Chloroform werden bei 0° 0,6 ml Thiophosphen in 5 ml Chloroform unter Rühren zugegeben. Nach 4-stündigem Rühren bei Raumtemperatur wird das Gemisch filtriert. Das Filtrat wird im Vakuum eingeengt und an einer Silicagel-Säule chromatographisch gereinigt. Als Eluierungsmittel wird Hexan/Methylenchlorid im Verhältnis 1:1 verwendet. Man erhält das 2-Tert-butyl-6-isothiocyanat-5-[thiomorpholin-4-yl]benzothiazol mit einem Schmelzpunkt von 155-159°.

Das Ausgangsmaterial für die obige Verbindung wird wie folgt erhalten:

Ein Gemisch aus 2 g 2-Tert-butyl-5-chlor-6-nitrobenzothiazol (beschrieben im Beispiel 5), 2 g getrocknetes Kaliumcarbonat und 1,5 ml Thiomorpholin in 7 ml 1-Methyl-2-pyrrolidinon wird in einem zugeschmolzenen Bombenrohr aus Glas 12 Stunden lang auf 120° erhitzt. Der Inhalt wird dann in Wasser geschüttet, der feste

Rückstand wird abfiltriert, mit Wasser und Isopropanol gewaschen. Man erhält das 2-Tert-butyl-6-nitro-5-[thiomorpholin-4-yl]benzothiazol mit einem Schmelzpunkt von 117-120°.

Eine Lösung aus 4,2 g der soeben erhaltenen Verbindung in 150 ml Methanol wird in Gegenwart von 2,5 g Raney Nickel bei 45° hydriert. Nach dem Entfernen des Katalysatorrückstandes wird die Lösung eingeengt, der feste Rückstand abfiltriert und mit kaltem Methanol gewaschen. Man erhält das 6-Amino-2-tert-butyl-5-[thiomorpholin-4-yl]benzothiazol mit einem Schmelzpunkt von 170-175°.

Beispiel 9: Ein Gemisch aus 2 g 6-Amino-2-tert-butyl-5-[4-phenylpiperazin-1-yl]benzothiazol und 2 g getrocknetes Kaliumcarbonat in 150 ml Chloroform wird mit 4 ml Thiophosgen bei 0° behandelt. Das Reaktionsgemisch wird bei Raumtemperatur weitere 3 Stunden gerührt und anschliessend noch unter Rühren (5 Minuten lang) mit einer 10%-igen Natronlauge versetzt. Die organische Schicht wird getrennt, getrocknet und eingeengt. Man erhält ein Oel, welches chromatographisch unter Verwendung einer 1% Methanol enthaltenden Chloroformlösung als Eluierungsmittel an einer Silicagel-Säule gereinigt wird. Man erhält das 2-Tert-butyl-6-isothiocyanat-5-[4-phenylpiperazin-1-yl]benzothiazol mit einem Schmelzpunkt von 120-123°.

Das Ausgangsmaterial der oben erhaltenen Verbindung wird wie folgt hergestellt:

Ein Gemisch aus 7 g 2-Tert-butyl-5-chloro-6-nitrobenzothiazol (beschrieben im Beispiel 5) in 8,2 g N-Phenyl-piperazin wird 12 Stunden lang auf eine Temperatur von 150-160° erhitzt, gekühlt und in Wasser geschüttet. Die erhaltene Paste wird mit Methylenchlorid extrahiert, mit 2N Salzsäurelösung und anschliessend noch mit einer 10%igen Natronlauge und Wasser gewaschen. Die organische Schicht wird getrocknet, das Lösungsmittel wird abgedampft und der erhaltene feste Rückstand wird aus Isopropylalkohol umkristallisiert. Man erhält das 2-Tert-butyl-6-nitro-5-[4-phenylpiperazin-1-yl]benzothiazol mit einem Schmelzpunkt von 152-155°.

Beispiel 10: Ein Gemisch aus 2 g 6-Amino-2-tert-butyl-5-[4-carbäthoxy-piperazin-1-yl]benzothiazol und 2 g Natriumbicarbonat in 200 ml Chloroform wird in einem Eisbad gekühlt und mit 4 ml Thiophosgen in 10 ml Chloroform versetzt und das Gemisch wird weitere 2 Stunden lang bei Raumtemperatur gerührt. Der feste Rückstand wird abfiltriert und das Filtrat wird eingeengt im Vakuum. Man erhält ein Oel, welches an einer Silicagel-Säule unter Verwendung einer 1% Methanol enthaltenden Chloroformlösung als Eluierungsmittel chromatographisch gereinigt wird. Man erhält 2-Tert-butyl-5-[4-carbäthoxy-piperazin-1-yl]-6-isothiocyanat-benzothiazol mit einem Schmelzpunkt von 131-134°.

Das Ausgangsmaterial der oben erhaltenen Verbindung wird wie folgt hergestellt.

Ein Gemisch aus 3,5 g 2-Tert-butyl-5-chloro-6-nitrobenzothiazol (beschrieben in Beispiel 5) und 5,8 g N-Carbäthoxypiperazin wird 2 Stunden lang auf 180° erhitzt, gekühlt und anschliessend in Wasser geschüttet. Der feste Rückstand wird abfiltriert, mit Wasser gewaschen und in Isopropylalkohol umkristallisiert. Man erhält das 2-Tert-butyl-5-(4-carbäthoxypiperazin-1-yl)-6-nitro-benzothiazol mit einem Schmelzpunkt von 100-102°.

Eine Lösung enthaltend 4,4 g der oben erhaltenen Verbindung in 150 ml Methanol wird in Gegenwart von 3 g Raney Nickel bei 45° hydriert. Der erhaltene feste Rückstand ergibt das 6-Amino-2-tert-butyl-5-[4-carbäthoxy-piperazin-1-yl]benzothiazol, welches bei einer Temperatur von 215-220° schmilzt.

Beispiel 11: Ein Gemisch aus 2 g 6-Amino-2-tert-butyl-5-(4-acetyl-piperazin-1-yl)benzothiazol und 1,5 g Natriumbicarbonat in 150 ml Chloroform wird auf eine Temperatur von 0° gekühlt und tropfenweise mit 4 ml Thiophosgen versetzt. Unter weiterem Rühren wird das Reaktionsgemisch über Nacht stehen gelassen. Der feste Rückstand wird abfiltriert und das Filtrat unter Vakuum eingeengt. Man erhält

ein Oel, welches an einer Silicagel-Säule unter Verwendung einer 1% Methanol enthaltenden Chloroformlösung als Eluierungsmittel chromatographisch gereinigt wird. Man erhält das 2-Tert-butyl-6-isothiocyanat-5-[4-acetylpiperazin-1-yl]benzothiazol, welches bei 100-102° schmilzt.

Das Ausgangsmaterial für die oben erhaltene Verbindung wird wie folgt hergestellt:

Eine Lösung aus 13,5 g 2-Tert-butyl-5-chlor-6-nitro-benzothiazol (beschrieben in Beispiel 5) und 40 g getrocknetes Piperazin in 100 ml Dimethylsulfoxid wird 2 Stunden lang auf 140° erhitzt, gekühlt und in Eiswasser geschüttet. Der gelbe feste Rückstand wird abfiltriert, mit Wasser gewaschen und bei 80° getrocknet. Man erhält das 2-Tert-butyl-6-nitro-5-[piperazin-1-yl]benzothiazol mit einem Schmelzpunkt von 144-146°.

Ein Gemisch aus 5 g der oben erhaltenen Verbindung und 4 ml Essigsäureanhydrid in 8 ml Eisessig wird 2 Stunden lang zum Rückfluss erhitzt, gekühlt und in Wasser geschüttet. Der erhaltene Rückstand wird abfiltriert, mit Wasser gewaschen und bei 80° getrocknet. Man erhält das 5-(4-Acetyl-piperazin-1-yl)-2-tert-butyl-6-nitro-benzothiazol mit einem Schmelzpunkt von 166-168°. Eine Lösung aus 3 g der oben erhaltenen Verbindung in 150 ml Methanol wird in Gegenwart von 2 g Raney Nickel hydriert. Nach dem Entfernen des Katalysators wird die Lösung eingeengt und man erhält als festen Rückstand 5-(4-Acetylpiperazin-1-yl)-6-amino-2-tert-butyl-benzothiazol mit einem Schmelzpunkt von 182-185°.

<u>Beispiel 12:</u> Ein Gemisch aus 1 g 6-Amino-2-tert-butyl-5-[4-methansulfonyl-piperazin-1-yl]benzothiazol und 50 g Natriumbicarbonat in 100 ml Chloroform wird in einem Eisbad gekühlt und mit 2 ml Thiophosgen in 10 ml Chloroform unter Rühren zugefügt. Das Gemisch wird über Nacht bei Raumtemperatur gerührt. Nach dem Abfiltrieren des festen Rückstandes wird das Filtrat im Vakuum eingeengt und der erhaltene Rückstand an einer Silicagel-Säule chromatographisch

gereinigt. Als Eluierungsmittel wird eine 1% Methanol enthaltende Chloroformlösung verwendet. Man erhält das 2-Tert-butyl-6-isothiocyanat-5-[4-methansulfonyl-piperazin-1-yl]benzothiazol mit einem Schmelzpunkt von 128-130°.

Das Ausgangsmaterial der oben erhaltenen Verbindung wird wie folgt erhalten:

Zu einer Lösung aus 5 g 2-Tert-butyl-6-nitro-5-[piperazin-1-yl]-benzothiazol (beschrieben in Beispiel 11) in 25 ml Pyridin werden 2,3 g Methansulfonylchlorid hinzugefügt und 10 Minuten lang auf 90° erhitzt. Das Gemisch wird gekühlt, in Wasser geschüttet und der feste Rückstand abfiltriert, mit Wasser und Isopropylalkohol gewaschen und man erhält 2-Tert-butyl-6-nitro-5-[4-methansulfonyl-piperazin-1-yl]benzothiazol mit einem Schmelzpunkt von 195-197°. Eine Lösung aus 5 g der oben erhaltenen Verbindung in 500 ml Methanol wird in Gegenwart von 3 g Raney Nickel bei 40° hydriert. Nach dem Abfiltrieren des Katalysators wird die Lösung eingeengt. Der erhaltene feste Rückstand wird abfiltriert und mit Hexan gewaschen. Man erhält 6-Amino-2-tert-butyl-5-[4-methansulfonyl-piperazin-1-yl]benzothiazol mit einem Schmelzpunkt von 153-155°.

Beispiel 13: Ein Gemisch aus 1,5 g N-[(2-Tert-butyl-5-N,N-dimethyl-amino)benzothiazol-6-yl]thioharnstoff und 0,4 g Ammoniumsulfat in 160 ml Chlorbenzol wird unter Stickstoff 12 Stunden lang zum Rückfluss erhitzt und anschliessend danach das Lösungsmittel im Vakuum entfernt. Der erhaltene feste Rückstand wird mit Hexan extrahiert und durch eine Silicagel-Säule filtriert. Man erhält das 2-Tert-butyl-5-(N,N-dimethylamino)-6-isothiocyanat-benzothiazol mit einem Schmelzpunkt von 63-66°.

Das Ausgangsmaterial wird wie folgt hergestellt:

Eine Lösung aus 16,2 g 2-Tert-butyl-5-chlor-6-nitro-benzothiazol (beschrieben in Beispiel 5), in 15 ml Hexamethylphosphortriamid wird für 10 Stunden auf 200° erhitzt und anschliessend in Eiswasser

geschüttet. Die flüssige Phase wird getrennt und mit Aether extrahiert, getrocknet über wasserfreiem Natrium und anschliessend wird der Aether unter Vakuum entfernt, wobei ein rot gefärbtes Oel erhalten wird, welches über eine Silicagel-Säule chromatographisch gereinigt wird. Nach Eluierung mit einem Benzol/Hexangemisch (1:1) erhält man 2-Tert-butyl-5-(N,N-dimethylamino)-6-nitro-benzothiazol mit einem Schmelzpunkt von 65-68°.

Eine Lösung enthaltend 12 g der oben erhaltenen Verbindung in 350 g Methanol wird in Gegenwart von 40 g Raney Nickel bei Raumtemperatur hydriert. Nach Entfernung des Katalysators wird die Lösung eingeengt und der erhaltene abfiltrierte Rückstand ergibt das 6-Amino-2-tert-butyl-5-(N,N-dimethylamino)-benzothiazol mit einem Schmelzpunkt von 169°.

Zu einem gerührten Gemisch aus 6,7 g der oben erhaltenen Verbindung und 4,1 g getrocknetem Ammoniumthiocyanat in 40 ml Isopropanol wird bei 70° unter Stickstoff 2,7 g konz. Schwefelsäure tropfenweise zugefügt. Das Gemisch wird 24 Stunden zum Rückfluss erhitzt, gekühlt, in Eiswasser geschüttet und die erhaltene Lösung wird anschliessend mit einer 30%igen Natronlauge alkalisch gestellt. Der erhaltene feste Rückstand wird abfiltriert, mit Wasser und Isopropanol gewaschen. Man erhält den N-[2-Tert-butyl-5-(N,N-dimethylamino)benzothiazol-6-yl]-thioharnstoff mit einem Schmelzpunkt von 275-280°.

Beispiel 14: Zu einem gekühlten Gemisch aus 1,5 g 6-Amino-2-tert-butyl-5-[3-methylpiperidin-1-yl]benzothiazol und 1,5 g Kaliumbicarbonat in 50 ml Chloroform wird tropfenweise 0,6 g Thiophosgen in 5 ml Chloroform unter Rühren zugefügt. Das Rühren wird weitere 30 Minuten lang fortgesetzt, wobei die Temperatur auf 0-2° gehalten wird. Der erhaltene feste Rückstand wird abfiltriert und das Filtrat wird im Vakuum eingeengt. Der so erhaltene feste Rückstand wird in Chloroform gelöst und durch eine Silicagel-Säule filtriert. Das

Filtrat wird eingeengt. Man erhält das 2-Tert-butyl-6-isothiocyanat-5-[3-methylpiperidin-1-yl]benzothiazol mit einem Schmelzpunkt von 47-52°.

Das Ausgangsmaterial wird wie folgt erhalten:

Eine Lösung aus 2,7 g 2-Tert-butyl-5-chlor-6-nitro-benzothiazol (beschrieben in Beispiel 5) in 25 ml Dimethylsulfoxid wird mit 2,6 g entwässertem Kaliumcarbonat und 1 g 3-Methylpiperidin 11 Stunden lang auf 140° erhitzt, gekühlt und in Wasser geschüttet. Das erhaltene Reaktionsprodukt wird in Chloroform gelöst und durch eine Silicagel-Säule filtriert. Das Filtrat wird im Vakuum eingeengt und man erhält das 2-Tert-butyl-5-(3-methylpiperidin-1-yl)-6-nitro-benzothiazol als Oel.

Eine Lösung enthaltend 1,7 g 2-Tert-butyl-5-(3-methylpiperidin-1-yl)-6-nitro-benzothiazol in 50 ml Methanol wird in Gegenwart von 0,5 g Raney Nickel bei Raumtemperatur hydriert. Nach dem Entfernen des Katalysators wird die Lösung eingeengt und man filtriert als festen Bestandteil das 6-Amino-2-tert-butyl-5-[3-methylpiperidin-1-yl]benzothiazol mit einem Schmelzpunkt von 108-112°.

<u>Beispiel 15:</u> Zu einem gekühlten Gemisch aus 2,1 g 6-Amino-2-tert-butyl-5-[4-methylpiperidin-1-yl]benzothiazol und 2,6 g Kaliumtricarbonat in 20 ml Chloroform wird 1,6 g Thiophosgen in 5 ml Chloroform unter Rühren hinzugefügt. Das Rühren wird weitere 2 Stunden lang fortgesetzt, wobei die Temperatur auf 0-2° gehalten wird. Der erhaltene feste Rückstand wird abfiltriert und das Filtrat wird im Vakuum eingeengt. Der so erhaltene feste Rückstand wird in Chloroform gelöst und durch eine Silicagel-Säule filtriert. Das so gereinigte Filtrat ergibt beim Einengen im Vakiuum das 2-Tert-butyl-6-isothiocyanat-5-[4-methylpiperidin-1-yl]benzothiazol mit einem Schmelzpunkt von 54-58°.

Das Ausgangsmaterial wird wie folgt erhalten.

Eine Lösung enthaltend 32,4 g 2-Tert-butyl-5-chlor-6-nitro-benzothiazol (beschrieben in Beispiel 6) in 300 ml Dimethylsulfoxid wird 6 Stunden lang mit 12,8 g 4-Methylpiperidin und 35,8 g entwässertem Kaliumcarbonat unter Rühren auf 140° erhitzt, gekühlt und anschliessend in Wasser geschüttet. Der erhaltene feste ausgefallene Rückstand wird in Chloroform gelöst und durch eine Silicagel-Säule filtriert. Das Filtrat wird eingeengt und man erhält das 2-Tert-butyl-5-(4-methylpiperidin-1-yl)-6-nitro-benzothiazol mit einem Schmelzpunkt von 116-120°.

Eine Lösung enthaltend 15,2 g 2-Tert-butyl-5-(4-methylpiperidin-1-yl)-6-nitro-benzothiazol in 600 ml Methanol wird in Gegenwart von 6,5 g Raney Nickel bei Raumtemperatur hydriert. Nach dem Entfernen des Katalysators wird die Lösung eingeengt und man erhält als festen Rückstand das 6-Amino-2-tert-butyl-5-[4-methyl-piperidin-1-yl]-benzothiazol mit einem Schmelzpunkt von 146-148°.

Patentansprüche für die Vertragsstaaten DE, GB, FR, CH, LI, NL, BE, SE, LU, IT

1. Benzazolderivate der allgemeinen Formel I

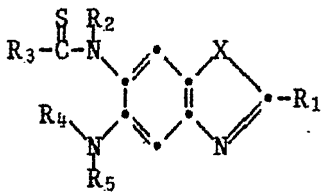

(I)

worin X Sauerstoff oder Schwefel, $R_1$ Niederalkyl, Niederalkenyl oder Cycloalkyl gegebenenfalls substituiert, $R_2$ Wasserstoff, und $R_3$ Niederalkylthio oder Niederalkenylthio gegebenenfalls substituiert durch eine veresterte Hydroxy-, Carboxy- oder veresterte Carboxygruppe oder $R_2$ und $R_3$ beide zusammengenommen eine zusätzliche Bindung in der C-N-Gruppierung, und $R_4$ und $R_5$ jeder für sich Wasserstoff, Niederalkyl oder Cycloalkyl oder beide zusammengenommen einen unsubstituierten oder substituierten bivalenten Kohlenwasserstoffrest aliphatischen Charakters, in welchem die Kohlenstoffatome der Kette durch ein Heteroatom unterbrochen sein können, bedeuten und ihre Salze.

2. Verbindungen der Formel I gemäss Anspruch 1, in der X Sauerstoff oder Schwefel, $R_1$ Niederalkyl oder Niederalkenyl, $R_2$ Wasserstoff, und $R_3$ gegebenenfalls durch Carboxy- oder veresterte Carboxygruppe substituiertes Niederalkylthio, oder $R_2$ und $R_3$ zusammengenommen eine zusätzliche Bindung in der C-N-Gruppierung, und $R_4$ und $R_5$ jeder für sich Wasserstoff oder Niederalkyl oder beide zusammengenommen einen unsubstituierten oder substituierten Niederalkylenrest, der gegebenenfalls durch ein Sauerstoff-, Schwefel- oder gegebenenfalls substituiertes Stickstoffatom unterbrochen sein kann mit 4-6 Kohlenstoffatomen in der Kette bedeuten und ihre Salze herstellt.

3. Verbindungen der Formel I gemäss Anspruch 1, in der X Schwefel, $R_1$ Niederalkyl, $R_2$ Wasserstoff und $R_3$ eine Carboxyniederalkylthiogruppe oder $R_2$ und $R_3$ zusammengenommen eine zusätzliche Bindung in der C-N-Gruppierung, und $R_4$ und $R_5$ jeder für sich Wasserstoff, Niederalkyl oder beide zusammen einen gegebenenfalls durch ein Sauerstoff-, Schwefel- oder Stickstoffatom, welches gegebenenfalls durch Niederalkyl, Niederalkanoyl, Niederalkoxycarbonyl, Niederalkansulfonyl oder Phenyl substituiert sein kann, unterbrochen sein kann, bedeuten und ihre Salze.

4. Verbindungen der Formel Ia,

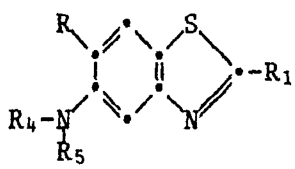

(Ia)

worin R die NCS oder $HN-\overset{S}{\overset{\|}{C}}-S(CH_2)_2COOH$-Gruppe, $R_1$ Niederalkyl mit 3 bis 4 C-Atomen und $R_4$ und $R_5$ jeder für sich Wasserstoff, Niederalkyl oder beide zusammen mit dem benachbarten Stickstoffatom einen Niederalkylenaminorest mit 4 oder 5 Kohlenstoffatomen in der Kette, der gegebenenfalls durch ein Sauerstoff-, Schwefel- oder auch gegebenenfalls durch Niederalkyl, Niederalkanoyl, Niederalkoxycarbonyl, Niederalkansulfonyl substituiertes Stickstoffatom unterbrochen sein kann, bedeuten und ihre pharmazeutisch verwendbaren Salze.

5. Verbindungen der Formel Ia, gemäss Anspruch 4, worin R die NCS oder $HN-\underset{S}{\underset{\|}{C}}-S(CH_2)_2-COOH$ Gruppe, $R_1$ tert-Butyl und $R_4$ und $R_5$ Niederalkyl, wie z.B. Methyl oder beide zusammen mit dem benachbarten Stickstoffatom als Niederalkylenamino einen heterocyclischen Rest, wie z.B. Pyrrolidino, Piperidino, Morpholino, Piperazino oder Methylpiperazino, Phenyl-piperazino, Acetylpiperazino, Methansulfonyl-piperazino, Methoxycarbonyl- oder Aethoxycarbonyl-piperazino bedeuten und ihre pharmazeutisch verwendbaren Salze.

6. 2-Tert-butyl-6-isothiocyanato-5-[piperidin-1-yl]benzothiazol und seine pharmazeutisch verwendbaren Salze.

7. 2-Tert-butyl-6-N-[(β-carboxyäthylthio)thiocarbonyl]-amino-5-[piperidin-1-yl]benzothiazol und seine pharmazeutisch verwendbaren Salze.

8. 2-Tert-butyl-6-isocyanat-5-[4-methyl-piperazin 1-yl]benzothiazol und seine pharmazeutisch verwendbaren Salze.

9. 2-Tert-butyl-6-N-[(β-carboxyäthylthio)thiocarbonyl]-amino-5-[4-methylpiperazin-1-yl]benzothiazol und seine pharmazeutisch verwendbaren Salze herstellt.

10. 2-Tert-butyl-6-isothiocyanat-5-[pyrrolidin-1-yl]benzothiazol und seine pharmazeutisch verwendbaren Salze.

11. 2-Tert-butyl-6-isothiocyanat-5-[morpholin-4-yl]benzothiazol und seine pharmazeutisch verwendbaren Salze.

12. 2-Tert-butyl-6-N-[(β-carboxyäthylthio)thiocarbonyl]-amino-5-[morpholin-4-yl]benzothiazol und seine pharmazeutisch verwendbaren Salze.

13. 2-Tert-butyl-6-isothiocyanat-5-[thiomorpholin-4-yl]benzothiazol und seine pharmazeutisch verwendbaren Salze.

14. 2-Tert-butyl-5-[4-carbäthoxy-piperazin-1-yl]-6-isothiocyanat-benzothiazol und seine pharmazeutisch verwendbaren Salze.

15. 2-Tert-butyl-6-isothiocyanat-5-[4-acetylpiperazin-1-yl]benzothiazol und seine pharmazeutisch verwendbaren Salze.

16. 2-Tert-butyl-6-isothiocyanat-5-[4-methansulfonyl-piperazin-1-yl]benzothiazol und seine pharmazeutisch verwendbaren Salze.

17. 2-Tert-butyl-5-(N,N-dimethylamino)-6-isothiocyanat-benzothiazol und seine pharmazeutisch verwendbaren Salze.

18. 2-Tert-butyl-6-isothiocyanat-5-[3-methylpiperidin-1-yl]benzothiazol und seine pharmazeutisch verwendbaren Salze.

19. 2-Tert-butyl-6-isothiocyanat-5-[4-methylpiperidin-1-yl]benzothiazol und seine pharmazeutisch verwendbaren Salze.

20. Pharmazeutische Präparate mit anthelminthischen Eigenschaften, gekennzeichnet durch einen Gehalt an einer unter der allgemeinen Formel I bzw. Formel Ia genannten Verbindungen gemäss Ansprüchen 1-19 in Kombination mit geeignetem Trägerstoff und/oder Verteilungsmittel.

21. Verwendung der Verbindungen der Ansprüche 1-19 zur Herstellung von pharmazeutischen Präparaten.

22. Verfahren zur Herstellung von Benzazolderivaten der in Anspruch 1 angegebenen allgemeinen Formel I, worin alle Symbole die im Anspruch 1 angegebenen Bedeutungen haben, und ihre Salze, dadurch gekennzeichnet, dass man

a) Verbindungen der Formel II

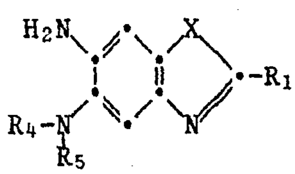

(II)

worin X, $R_1$, $R_4$ und $R_5$ die unter der Formel I angegebenen Bedeutungen haben mit Thiophosgen in einem inerten Lösungsmittel umsetzt, oder

b) eine Verbindung der Formel III

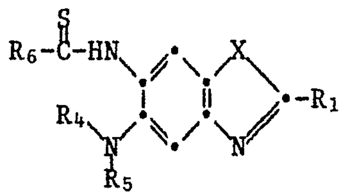

(III)

worin X, $R_1$, $R_4$ und $R_5$ die unter der Formel I angegebene Bedeutung haben und $R_6$ eine durch Pyrolyse abspaltbare Gruppe, vorzugsweise Amino, Niederalkylthio oder die Thioammoniumgruppe $^{\oplus}NH_4-\overset{\ominus}{S}-$ bedeutet, pyrolysiert, oder

c) eine Verbindung der Formel IV

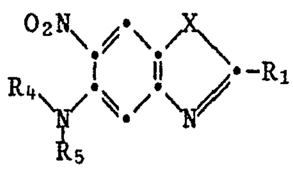

(IV)

worin X, $R_1$, $R_4$ und $R_5$ die oben angegebenen Bedeutungen haben, mit Schwefelkohlenstoff umsetzt, oder

d) eine Verbindung der Formel V

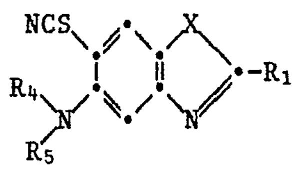

(V)

worin X, $R_1$, $R_4$ und $R_5$ die oben angegebenen Bedeutungen haben in einem hochsiedenden Lösungsmittel thermisch isomerisiert, und, wenn erwünscht, eine erhaltene Verbindung der Formel I in eine andere Verbindung der Formel I überführt, und/oder, wenn erwünscht, eine erhaltene freie Verbindung in ein Salz überführt.

0175650

23. Die nach dem Verfahren des Anspruchs 22 erhältlichen Verbindungen.

FO 7.4 HFO/hc*

Patentansprüche für den Vertragsstaat AT

1. Verfahren zur Herstellung von Benzazolderivaten der allgemeinen Formel I

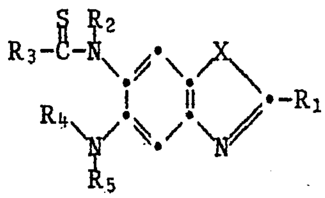

(I)

worin X Sauerstoff oder Schwefel, $R_1$ Niederalkyl, Niederalkenyl oder Cycloalkyl gegebenenfalls substituiert, $R_2$ Wasserstoff, und $R_3$ Niederalkylthio oder Niederalkenylthio gegebenenfalls substituiert durch eine veresterte Hydroxy-, Carboxy- oder veresterte Carboxygruppe oder $R_2$ und $R_3$ beide zusammengenommen eine zusätzliche Bindung in der C-N-Gruppierung, und $R_4$ und $R_5$ jeder für sich Wasserstoff, Niederalkyl oder Cycloalkyl oder beide zusammengenommen einen unsubstituierten oder substituierten bivalenten Kohlenwasserstoffrest aliphatischen Charakters, in welchem die Kohlenstoffatome der Kette durch ein Heteroatom unterbrochen sein können, bedeuten und ihre Salze, dadurch gekennzeichnet, dass man

a) Verbindungen der Formel II

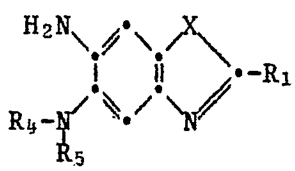

(II)

worin X, $R_1$, $R_4$ und $R_5$ die unter der Formel I angegebenen Bedeutungen haben mit Thiophosgen in einem inerten Lösungsmittel umsetzt, oder

b) eine Verbindung der Formel III

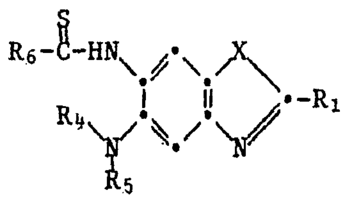

(III)

worin X, $R_1$, $R_4$ und $R_5$ die unter der Formel I angegebene Bedeutung haben und $R_6$ eine durch Pyrolyse abspaltbare Gruppe, vorzugsweise Amino, Niederalkylthio oder die Thioammoniumgruppe $^{\oplus}NH_4\text{-}\overset{\ominus}{S}\text{-}$ bedeutet, pyrolysiert, oder

c) eine Verbindung der Formel IV

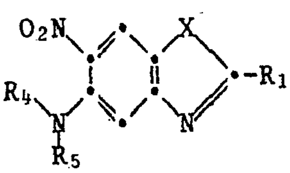

(IV)

worin X, $R_1$, $R_4$ und $R_5$ die oben angegebenen Bedeutungen haben, mit Schwefelkohlenstoff umsetzt, oder

d) eine Verbindung der Formel V

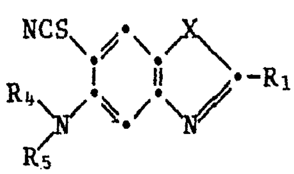

(V)

worin X, $R_1$, $R_4$ und $R_5$ die oben angegebenen Bedeutungen haben in einem hochsiedenden Lösungsmittel thermisch isomerisiert, und, wenn erwünscht, eine erhaltene Verbindung der Formel I in eine andere Verbindung der Formel I überführt, und/oder, wenn erwünscht, eine erhaltene freie Verbindung in ein Salz überführt.

2. Verfahren nach Anspruch 1b) zur Herstellung von Verbindungen der allgemeinen Formel I, dadurch gekennzeichnet, dass man die Pyrolyse oxydativ ausführt.

3. Verfahren nach Patentanspruch 1, dadurch gekennzeichnet, dass man Verbindungen der Formel I, in der X Sauerstoff oder Schwefel, $R_1$ Niederalkyl oder Niederalkenyl, $R_2$ Wasserstoff, und $R_3$ gegebenenfalls durch Carboxy- oder veresterte Carboxygruppe substituiertes Niederalkylthio, oder $R_2$ und $R_3$ zusammengenommen eine zusätzliche Bindung in der C-N-Gruppierung, und $R_4$ und $R_5$ jeder für sich Wasserstoff oder Niederalkyl oder beide zusammengenommen einen unsubstituierten oder substituierten Niederalkylenrest, der gegebenenfalls durch ein Sauerstoff-, Schwefel- oder gegebenenfalls substituiertes Stickstoffatom unterbrochen sein kann mit 4-6 Kohlenstoffatomen in der Kette bedeuten und ihre Salze herstellt.

4. Verfahren nach Patentanspruch 1, dadurch gekennzeichnet, dass man Verbindungen der Formel I, in der X Schwefel, $R_1$ Niederalkyl, $R_2$ Wasserstoff und $R_3$ eine Carboxyniederalkylthiogruppe oder $R_2$ und $R_3$ zusammengenommen eine zusätzliche Bindung in der C-N-Gruppierung, und $R_4$ und $Ra_5$ jeder für sich Wasserstoff, Niederalkyl oder beide zusammen einen gegebenenfalls durch ein Sauerstoff-, Schwefel- oder Stickstoffatom, welches gegebenenfalls durch Niederalkyl, Niederalkanoyl, Niederalkoxycarbonyl, Niederalkansulfonyl oder Phenyl substituiert sein kann, unterbrochen sein kann, bedeuten und ihre Salze herstellt.

5. Verfahren nach Patentanspruch 1, dadurch gekennzeichnet, dass man Verbindungen der Formel Ia,

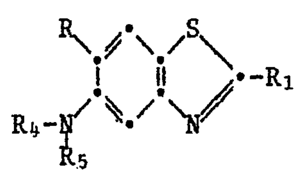

(Ia)

worin R die NCS oder $HN-\overset{S}{\overset{\|}{C}}-S(CH_2)_2COOH$-Gruppe, $R_1$ Niederalkyl mit 3 bis 4 C-Atomen und $R_4$ und $R_5$ jeder für sich Wasserstoff, Niederalkyl oder beide zusammen mit dem benachbarten Stickstoffatom einen Niederalkylenaminorest mit 4 oder 5 Kohlenstoffatomen in der Kette, gegebenenfalls durch ein Sauerstoff-, Schwefel- oder auch gegebenenfalls durch Niederalkyl, Niederalkanoyl, Niederalkoxycarbonyl, Niederalkansulfonyl substituierten Stickstoffatom unterbrochen sein kann, bedeuten und ihre pharmazeutisch verwendbaren Salze herstellt.

6. Verfahren nach Patentanspruch 1, dadurch gekennzeichnet, dass man Verbindungen der Formel Ia, gemäss Anspruch 5, worin R die NCS oder $HN-\underset{\underset{S}{\|}}{C}-S(CH_2)_2-COOH$ Gruppe, $R_1$ tert-Butyl und $R_4$ und $R_5$ Niederalkyl

Niederalkyl, wie z.B. Methyl oder beide zusammen mit dem benachbarten Stickstoffatom als Niederalkylenamino einen heterocyclischen Rest, wie z.B. Pyrrolidino, Piperidino, Morpholino, Piperazino oder Methylpiperazino, Phenyl-piperazino, Acetylpiperazino, Methansulfonyl-piperazino, Methoxycarbonyl- oder Aethoxycarbonyl-piperazino bedeuten und ihre pharmazeutisch verwendbaren Salze herstellt.

7. Verfahren nach Patentanspruch 1, dadurch gekennzeichnet, dass man 2-Tert-butyl-6-isothiocyanato-5-[piperidin-1-yl]benzothiazol und seine pharmazeutisch verwendbaren Salze herstellt.

8. Verfahren nach Patentanspruch 1, dadurch gekennzeichnet, dass man 2-Tert-butyl-6-N-[(β-carboxyäthylthio)thiocarbonyl]amino-5-[piperidin-1-yl]benzothiazol und seine pharmazeutisch verwendbaren Salze herstellt.

9. Verfahren nach Patentanspruch 1, dadurch gekennzeichnet, dass man 2-Tert-butyl-6-isocyanat-5-[4-methyl-piperazin-1-yl]benzothiazol und seine pharmazeutisch verwendbaren Salze herstellt.

10. Verfahren nach Patentanspruch 1, dadurch gekennzeichnet, dass man 2-Tert-butyl-6-N-[(β-carboxyäthylthio)thiocarbonyl]amino-5-[4-methyl-piperazin-1-yl]benzothiazol und seine pharmazeutisch verwendbaren Salze herstellt.

11. Verfahren nach Patentanspruch 1, dadurch gekennzeichnet, dass man 2-Tert-butyl-6-isothiocyanat-5-[pyrrolidin-1-yl]benzothiazol und seine pharmazeutisch verwendbaren Salze herstellt.

12. Verfahren nach Patentanspruch 1, dadurch gekennzeichnet, dass man 2-Tert-butyl-6-isothiocyanat-5-[morpholin-4-yl]benzothiazol und seine pharmazeutisch verwendbaren Salze herstellt.

13. Verfahren nach Patentanspruch 1, dadurch gekennzeichnet, dass man 2-Tert-butyl-6-N-[(β-carboxyäthylthio)thiocarbonyl]amino-5-[morpholin-4-yl]benzothiazol und seine pharmazeutisch verwendbaren Salze herstellt.

14. Verfahren nach Patentanspruch 1, dadurch gekennzeichnet, dass man 2-Tert-butyl-6-isothiocyanat-5-[thiomorpholin-4-yl]benzothiazol und seine pharmazeutisch verwendbaren Salze herstellt.

15. Verfahren nach Patentanspruch 1, dadurch gekennzeichnet, dass man 2-Tert-butyl-5-[4-carbäthoxy-piperazin-1-yl]-6-isothiocyanat-benzothiazol und seine pharmazeutisch verwendbaren Salze herstellt.

16. Verfahren nach Patentanspruch 1, dadurch gekennzeichnet, dass man 2-Tert-butyl-6-isothiocyanat-5-[4-acetylpiperazin-1-yl]benzothiazol und seine pharmazeutisch verwendbaren Salze herstellt.

17. Verfahren nach Patentanspruch 1, dadurch gekennzeichnet, dass man 2-Tert-butyl-6-isothiocyanat-5-[4-methansulfonyl-piperazin-1-yl]benzothiazol und seine pharmazeutisch verwendbaren Salze herstellt.

18. Verfahren nach Patentanspruch 1, dadurch gekennzeichnet, dass man 2-Tert-butyl-5-(N,N-dimethylamino)-6-isothiocyanat-benzothiazol und seine pharmazeutisch verwendbaren Salze herstellt.

19. Verfahren nach Patentanspruch 1, dadurch gekennzeichnet, dass man 2-Tert-butyl-6-isothiocyanat-5-[3-methylpiperidin-1-yl]benzothiazol und seine pharmazeutisch verwendbaren Salze herstellt.

20. Verfahren nach Patentanspruch 1, dadurch gekennzeichnet, dass man 2-Tert-butyl-6-isothiocyanat-5-[4-methylpiperidin-1-yl]benzothiazol und seine pharmazeutisch verwendbaren Salze herstellt.

FO 7.4 HFO/hc*